# EUROPÄISCHE PATENTSCHRIFT

(11) **EP 4 282 372 B1**
(45) Veröffentlichungstag und Bekanntmachung des Hinweises auf die Patenterteilung: **18.02.2026**
(21) Anmeldenummer: 23202322.6
(22) Anmeldetag: 17.12.2019
(51) Int. Cl.: A61C 8/00, A61B 17/88, B25B 23/142, B25B 13/46, A61B 90/00, A61C 1/18

(54) **DENTALMEDIZINISCHER DREHMOMENTSCHLÜSSEL**
DENTAL TORQUE WRENCH
CLÉ DYNAMOMÉTRIQUE DENTAIRE

(30) Priorität: 19.12.2018 EP 18214056
(43) Veröffentlichungstag der Anmeldung: 29.11.2023
(62) Teilanmeldung aus: 19818156.2
(73) Patentinhaber: Straumann Holding AG, 4002 Basel (CH)
(72) Erfinder: BRECHBÜHL, Tobias, 4052 Basel (CH); HEDINGER, Alain, 4052 Basel (CH)
(74) Vertreter: Schaad, Balass, Menzl & Partner AG

(56) Entgegenhaltungen:
- WO-A1-2009/036943
- DE-A1- 102017 000 222
- DE-B3- 102012 101 050
- US-A1- 2010 304 330
- US-B1- 6 988 430

## Beschreibung

Die Erfindung betrifft einen Drehmomentschlüssel als Ratscheninstrument für die Medizinaltechnik, insbesondere die Dentalmedizin, gemäss dem Oberbegriff des Anspruchs 1.

Aus der Dentalmedizin ist bekannt, Implantate in den Kieferknochen einzusetzen und an den Implantaten Verbindungselemente, wie Abutments, zu befestigen, auf welchen dann die Suprastruktur, insbesondere eine Krone oder eine Brücke, aufgesetzt wird. Zum Einschrauben des Implantats bzw. des Verbindungselements kann ein Eindrehinstrument auf dessen freies Ende formschlüssig aufgesetzt und das Eindrehinstrument über einen Drehmomentschlüssel gedreht werden. Langzeitstabilität und Zuverlässigkeit der Verschraubungen hängen davon ab, dass letztere mit dem jeweils optimalen Drehmoment ausgeführt werden. Ein zu schwaches Anziehen kann später zur Lockerung der Verschraubungen führen, während ein zu starkes Anziehen die eingesetzten Verbindungselemente und das Implantat überlastet und deren Bruchgefahr erhöht bzw. Schäden am Knochen verursacht.

Ein Drehmomentschlüssel mit einer Ratschenfunktion zur Anwendung in der Dentalmedizin ist in der EP 0 704 281 A1 offenbart. Der Drehmomentschlüssel umfasst ein Drehmomentinstrument, insbesondere ein Ratscheninstrument, sowie einen daran als Zubehör ansetzbaren Drehmomentindikator. Der Drehmomentindikator besitzt einen hülsenförmigen, auf einen Griff des Drehmomentinstruments aufschiebbaren Träger, an dem ein elastischer Biegestab befestigt ist. Auf das freie Ende des Biegestabes wird bei Betätigung eine Kraft in Anzugsrichtung des Drehmomentschlüssels ausgeübt und das erzeugte Drehmoment an einer Masseinteilung angezeigt. Die Reinigung und die Sterilisation dieses Drehmomentschlüssels erfordern eine Demontage des Drehmomentschlüssels und sind somit relativ aufwendig. Ferner ist seine Herstellung aufgrund der Vielzahl der Bauteile relativ teuer.

Aus der DE 20 2004 014 195 U1 ist ein Drehmomentschlüssel als Ratscheninstrument für die Medizinaltechnik bekannt, welcher einen zuvorderst liegenden Kopfbereich, einen anschliessenden Halsbereich, dem ein Schaftbereich folgt, und einen zuhinterst angeordneten Griffbereich umfasst. Der Kopfbereich, der Halsbereich, der Schaftbereich und der Griffbereich erstrecken sich in einer Ebene. Der Drehmomentschlüssel umfasst ferner eine im Kopfbereich vorgesehene Aufnahmeöffnung, die von einer Umfassung umgeben ist und einen Mittelpunkt besitzt, durch den sich eine Achse senkrecht zur Ebene erstreckt. Die Aufnahmeöffnung dient zum Einsetzen eines Eindrehinstruments in der Erstreckung der Achse. Weiter umfasst der Drehmomentschlüssel ein an der Peripherie der Aufnahmeöffnung angeordnetes, begrenzt bewegliches Klinkensegment, dessen Frontpartie zur Aufnahmeöffnung weist. Die Frontpartie ist dazu bestimmt, bei Betätigung des Drehmomentschlüssels im Einschraubmodus, mit einer am Kopf des Eindrehinstruments vorhandenen Aussenkontur in mitnehmenden Eingriff zu kommen, und bei Betätigung des Drehmomentschlüssels in Rückwärtsrichtung, also im Ratschenmodus, den mitnehmenden Eingriff mit der am Kopf des Eindrehinstruments vorhandenen Aussenkontur zu lösen. Weiter umfasst der Drehmomentschlüssel einen vom Halsbereich längs des Schaftbereichs verlaufenden, biegesteifen Basisast. Vom Klinkensegment erstreckt sich eine Klinkenfeder in den Halsbereich hinein, wobei Klinkensegment und Klinkenfeder eine einstückige Klinke bilden. Beiderseits der Klinke sind lange dünne Schlitze ausgebildet, welche die Auslenkung der Klinke in der Ebene gegen die Kraft der Klinkenfeder erlauben. Ferner ist die Klinke einstückig aus dem Halsbereich herausgeformt. Durch die einteilige Ausführung dieses Drehmomentschlüssels fällt die Demontage bei der Reinigung und Sterilisation weg. Allerdings erschweren die langen dünnen Schlitze beiderseits der Klinke die Reinigung des Drehmomentschlüssels.

WO 2009/036943 A1 offenbart ein Schraubwerkzeug mit einem Ratschenkopf und einem Betätigungsgriff für die Anwendung in der Medizinaltechnik, insbesondere der Dentalprothetik. Der Ratschenkopf weist eine Aufnahmeöffnung auf, die von einer Umfassung umgeben ist. Die Umfassung ist zur Aufnahme eines Eindrehwerkzeugs ringförmig ausgebildet und weist an einer Stelle in Umfangsrichtung eine Unterbrechung auf. Im Bereich dieser Unterbrechung ist auf einer Seite der Umfassung eine Klinke und auf der anderen Seite der Betätigungsgriff angeordnet. Klinke und Umfassung sind einstückig ausgeführt, wobei die Umfassung die Federung der Klinke übernimmt. Die Federkraft wird dabei durch die Materialstärke der Umfassung sowie durch die Materialwahl festgelegt.

In einer Ausführungsform offenbart DE 10 2012 101050 B3 eine einstückige Dentalratsche umfassend ein Griffstück und ein Gehäuse zur Aufnahme eines Eindrehwerkzeugs. Das Gehäuse ist als biegeelastische Spirale mit zwei Umdrehungen ausgebildet. US 2010/304330 A1 offenbart einen Drehmomentschlüssel umfassend einen Griff und ein Kopfstück, das zur lösbaren Aufnahme eines Werkzeugstücks ausgebildet ist. Der Griff ist als ein längliches Körperelement ausgebildet und umfasst einen biegbaren Drehmomentarm zur Angabe des auf den Drehmomentarm aufgebrachten Drehmoments. Ferner umfasst der Griff ein Anzeigeelement mit einer Anzeige und ein Ratschenelement, das dazu ausgebildet ist, mit dem Werkzeugstück in Eingriff zu gehen.

Aufgabe der vorliegenden Erfindung ist es somit, einen Drehmomentschlüssel für die Medizinaltechnik, insbesondere für die Dentalmedizin, zur Verfügung zu stellen, welche günstig herstellt und einfach gereinigt werden kann.

Diese Aufgabe wird erfindungsgemäss durch ein Drehmomentschlüssel gemäss Anspruch 1 gelöst. Bevorzugte Ausführungsformen der Erfindung sind in den abhängigen Ansprüchen wiedergegeben.

Die Erfindung betrifft einen Drehmomentschlüssel als Ratscheninstrument für die Medizinaltechnik, insbesondere die Dentalmedizin. Der Drehmomentschlüssel umfasst einen eine Aufnahmeöffnung aufweisenden Kopfbereich, einen an den Kopfbereich anschliessenden Halsbereich, einen am Halsbereich befestigten, sich wenigstens annähernd in einer Ebene, vorzugsweise in der Ebene erstreckenden, stabförmigen Betätigungshebel zum Aufbringen eines Drehmoments auf den Kopfbereich, und eine die Aufnahmeöffnung des Kopfbereichs bildende Umfassung. Der Halsbereich bildet ein wenigstens annähernd quaderförmiges, vorzugsweise massives Teil. Die Umfassung definiert eine wenigstens annähernd rechtwinklig, vorzugsweise rechtwinklig zur Ebene verlaufende Rotationsachse und ist dazu bestimmt, ein Eindrehinstrument in der Erstreckung der Rotationsachse aufzunehmen. Die Ebene kann eine Vorderseite des Drehmomentschlüssels und eine parallel zur Ebene verlaufende weitere Ebene kann eine Rückseite des Drehmomentschlüssels umfassen.

Ferner umfasst der Drehmomentschlüssel einen Federbügel, und eine an diesem ausgebildeten Rastnase, welche in ihrer Ruhestellung in die Aufnahmeöffnung hineinragt. Die Ruhestellung ist die Position der Rastnase, wenn kein Eindrehinstrument in die Aufnahmeöffnung eingesetzt ist und keine Kraft in radialer Richtung auf den Federbügel ausgeübt wird.

Die Rastnase weist einen Mitnahmeabschnitt auf, welcher dazu bestimmt ist, bei in die Aufnahmeöffnung eingesetztem Eindrehinstrument, bei einer Drehung in Anzugsrichtung des Betätigungshebels in einer Mitnahmestellung der Rastnase mit einer Gegenfläche des Eindrehinstruments zusammenzuwirken. Die Mitnahmestellung kann der Ruhestellung entsprechen, wenn der Federbügel wenigstens annähernd keine Kraft gegen das eingesetzte Eindrehinstrument ausübt. Beim Zusammenwirken des Mitnahmeabschnitts mit der Gegenfläche wird ein Drehmoment durch eine formschlüssige Mitnahme ans Eindrehinstrument übertragen.

Die Rastnase ist dazu bestimmt, bei einer Drehung entgegen der Anzugsrichtung, gegen die Kraft des Federbügels sich in radialer Richtung gegen aussen in eine Freigabestellung zu bewegen, um einen Freilauf zwischen dem Kopfbereich und dem Eindrehinstrument zu bilden.

Erfindungsgemäss weist die Umfassung eine über ihren gesamten Querschnitt verlaufende Durchbrechung auf und bildet die Umfassung den Federbügel.

In einer bevorzugten Ausführungsform ist die Rastnase integral und einstückig mit dem Federbügel ausgebildet. Dies vereinfacht die Reinigung, weil eine Schmutzablagerung an einer Verbindungsstelle zwischen der Rastnase und dem Federbügel verhindert wird.

In einer bevorzugten Ausführungsform ist der Mitnahmeabschnitt durch eine wenigstens annähernd radial zur Rotationsachse verlaufende Mitnahmefläche gebildet, welche dazu bestimmt ist, mit der vorzugsweise wenigstens annähernd radial zur Rotationsachse verlaufenden Gegenfläche des eingesetzten Eindrehinstruments zusammenzuwirken. Diese Ausbildung der Mitnahmefläche und der Gegenfläche ermöglicht eine sichere Mitnahme des Eindrehinstruments.

Ferner kann die radial zur Rotationsachse gemessene Länge der Mitnahmefläche, welche Mitnahmefläche in Kontakt mit der Gegenfläche des Eindrehinstruments kommt, hier als Arbeitslänge bezeichnet, ebenfalls als Parameter verwendet werden, um eine sichere Mitnahme des Eindrehinstruments zu gewährleisten. Die Arbeitslänge entspricht der Länge der benötigten Bewegung der Rastnase, bezüglich der Rotationsachse radial nach aussen, sodass die Rastnase ihre Freigabestellung erreichen kann. Eine kurze Arbeitslänge kann folglich von Vorteil sein, weil die resultierende Verformung des Federbügels entsprechend klein ist und somit bleibt die Verformung im elastischen Bereich des Federbügels. Bei der Verwendung des Drehmomentschlüssels kann jedoch eine zu kurze Arbeitslänge dazu führen, dass der Mitnahmeabschnitt wegen einer unerwünschten Bewegung des Drehmomentschlüssels bezüglich des Eindrehinstruments auf die Gegenfläche um die Arbeitslänge bezüglich der Rotationsachse radial nach aussen rutscht, und folglich, dass die Rastnase aus der Mitnahmestellung ausrastet. Vorzugsweise ist die radiale Länge der Mitnahmefläche grösser als oder gleich wie die radiale Länge der Gegenfläche, um die Kraftübertragung zu optimieren. Vorzugsweise bewegt sich die radiale Länge der Mitnahmefläche zwischen 0,2 mm und 5 mm, um die Kraftübertragung zu optimieren.

In einer bevorzugten Ausführungsform grenzt die Rastnase an die Durchbrechung an. Besonders bevorzugt grenzt die Rastnase an die Durchbrechung direkt an. Somit ist der Umschlingungswinkel des vorliegenden Federbügels um das eingesetzte Eindrehinstrument herum maximiert. Durch die Umschlingung entstehen zusätzlich Haftreibungskräfte in Umfangsrichtung zwischen dem Federbügel und dem eingesetzten Eindrehinstrument, die das Halten des Eindrehinstruments in die Aufnahmeöffnung unterstützen. Dies bewirkt, dass die in Umfangsrichtung auf den Mitnahmeabschnitt der Rastnase wirkende Kraft beim Drehen in Anzugsrichtung kleiner ist.

In einer bevorzugten Ausführungsform weist die Rastnase eine, in Anzugsrichtung gesehen, der Mitnahmefläche nachlaufende, vorzugsweise an die Mitnahmefläche anschliessende Angriffsfläche auf, welche die Bewegung der Rastnase in die Freigabestellung und folglich den Freilauf erlaubt. Bei der Drehung des Betätigungshebels entgegen der Anzugsrichtung bildet die Angriffsfläche die Fläche, über welche die Kraft des Federbügels auf das Eindrehinstrument ausgeübt wird.

Besonders bevorzugt ist die Angriffsfläche durch eine Abschrägung gebildet, deren Abstand zur Rotationsachse entgegen der Anzugsrichtung zunimmt und welche den Freilauf erlaubt. Vorzugsweise nimmt der Abstand ohne Unterbrechung zu, um einen kontinuierlichen, eine gleichbleibende Reibung der Angriffsfläche auf das Eindrehinstrument aufweisenden Freilauf zu ermöglichen. Somit erfüllt der Drehmomentschlüssel ruckfrei seine Ratschenfunktion.

In einer besonders bevorzugten Ausführungsform erstreckt sich die Abschrägung mindestens annähernd bis zum freien Ende des Federbügels. Dies ermöglicht eine einfache Herstellung der der Rotationsache zugewandten Umfangsfläche des Federbügels.

Besonders bevorzugt weist die Angriffsfläche eine, in Anzugsrichtung gesehen, der Mitnahmefläche nachlaufende, in Umfangsrichtung verlaufende Verlängerungsfläche auf, welche an die Mitnahmefläche direkt anschliesst, und an welche Verlängerungsfläche die Abschrägung anschliesst. Die Verlängerungsfläche und die Abschrägung begrenzen ein Volumen der Rastnase, welches dazu dient, die Abscherung des Mitnahmeabschnitts im Anwendungsfall zu verhindern. Durch dieses Volumen kann gegebenenfalls der Verschleiss des Eindrehinstruments durch die Rastnase beim Freilauf reduziert werden, weil die Rastnase keine scharfe Kante, zwischen der Mitnahmefläche und der Abschrägung aufweist.

In einer besonders bevorzugten Ausführungsform weist die Abschrägung eine Länge, in radialer Richtung gemessen, gleich wie oder grösser als die Arbeitslänge. Dadurch wird vermieden, dass die Bewegung der Rastnase in die Freigabestellung bei der Drehung entgegen der Anzugsrichtung durch das Eindrehinstrument blockiert wird.

In einer besonders bevorzugten Ausführungsform ist ein Winkel, in der Ebene E gemessen, zwischen der Abschrägung und einer Tangente zur Umfangsrichtung, wobei die Tangente an der Schnittstelle der Abschrägung und der Umfangsrichtung verläuft, kleiner als 45°, bevorzugt zwischen 15° und 45°, besonders bevorzugt zwischen 25° und 40°. Der Winkel ist kleiner als 45°, sodass die Bewegung der Rastnase in die Freigabestellung bei der Drehung entgegen der Anzugsrichtung wenig Raum in radialer Richtung benötigt. Insbesondere im Mundbereich kann die Bewegung der Rastnase nämlich eingeschränkt sein. Die Reibungskraft zwischen der Oberfläche der Abschrägung und dem Eindrehinstrument liegt bei einem Winkel zwischen 15° und 45° in einem Bereich, in dem das durch Reibung ans Eindrehinstrument übertragene Drehmoment bei der Drehung entgegen der Anzugsrichtung, z.B. das Freilaufmoment, unter einem Lösedrehmoment des Implantats oder des Verbindungselements bleibt. Dies ist insbesondere wichtig bei der Implantation in Knochen, der instabil und fragil sein kann, womit das Lösedrehmoment klein sein sollte. Vorzugsweise ist das Freilaufmoment kleiner als 3Ncm. Erfahrungsgemäss ist ein Winkel zwischen 25° und 40° optimal, um das Freilaufmoment und gleichzeitig die radiale Bewegung der Rastnase in die Freigabestellung zu optimieren.

In einer bevorzugten Ausführungsform erstreckt sich die Rastnase, in axialer Richtung gemessen, über die gesamte Dicke der Umfassung. Die Erstreckung in axialer Richtung der Rastnase ermöglicht eine sichere Mitnahme, da die Mitnahmefläche entsprechend grösser ausfällt. Es ist jedoch auch möglich, dass die Rastnase sich nur über einen Teil der gesamten Dicke erstreckt, vorzugsweise, in axialer Richtung gesehen, zentriert bezüglich der Dicke der Umfassung. Durch die zentrische Lage der Rastnase gibt es keine lateralen Biegemomente auf den Federbügel.

Ein herkömmliches Eindrehinstrument umfasst einen zylindrischen, eine Drehachse des Eindrehinstruments definierenden Ratschenkopf, welcher in die Aufnahmeöffnung des Drehmomentschlüssels eingesetzt werden kann, und einen an den Ratschenkopf anschliessenden, sich in Richtung der Drehachse erstreckenden Schaft. Das freie Ende des Schaftes weist ein vorzugsweise standardisiertes Profil, beispielsweise einen Profil torx^{®} oder einen sechskantigen Kopf, zum Zusammenwirken mit dem Implantat bzw. dem Verbindungselement auf, sodass das Eindrehinstrument das auf den Ratschenkopf aufgebrachte Drehmoment an das Implantat bzw. das Verbindungselement übertragen kann. Der Ratschenkopf weist eine Umfangsfläche auf, welche eine die Gegenfläche bildende Konturierung umfasst, um eine Übertragung des auf den Drehmomentschlüssel aufgebrachten Drehmoments an das Eindrehinstrument zu ermöglichen. Die Konturierung kann von einer vorzugsweise parallel zur Drehachse des Eindrehinstruments verlaufenden Nut gebildet sein.

Vorzugsweise weist die Nut einen mindestens annähernd rechteckigen Querschnitt auf, deren in Anzugsrichtung bezüglich der Mitnahmefläche vorlaufende Wand bevorzugt wenigstens annähernd radial in Richtung zur Drehachse verläuft und die Gegenfläche bildet.

Vorzugsweise umfasst die Konturierung eine Mehrzahl von Nuten, welche gleich ausgebildet und, in Umfangsrichtung gesehen, im gleichen Abstand zueinander angeordnet sind.

In einer bevorzugten Ausführungsform des Drehmomentschlüssels ist die Rastnase so ausgebildet, dass, in Umfangsrichtung der Umfassung gemessen, die Gesamtlänge der Abschrägung gegebenenfalls zusammen mit der Verlängerungsfläche länger als die Breite der Nut ist. Somit kann das Einschieben der ganzen Rastnase in die Nut und die daraus resultierende mögliche Blockierung der Bewegung der Rastnase in die Freigabestellung bei einer Drehung entgegen der Anzugsrichtung wenigstens annähernd vollständig vermieden werden.

In einer bevorzugten Ausführungsform weist der Federbügel auf seiner der Rotationsachse zugewandten Seite eine, in Anzugsrichtung gesehen, bezüglich der Mitnahmefläche vorlaufende, an die Mitnahmefläche anschliessende Ausnehmung auf. Vorzugsweise ist die Ausnehmung wenigstens annähernd halbkreisförmig. Ferner ist sie so ausgebildet, dass ein der Gegenfläche vorlaufende Teil des Ratschenkopfs in die Ausnehmung bei einer Drehung in Anzugsrichtung hineinragen kann. Somit wird eine sichere Mitnahme des Eindrehinstruments sichergestellt.

In einer bevorzugten Ausführungsform grenzt die Durchbrechung an den Halsbereich an. Vorzugsweise ist die Durchbrechung durch einen sich wenigstens annähernd radial zur Rotationsachse erstreckenden Schlitz gebildet. Somit ist die Länge des Federbügels, in Umfangsrichtung gemessen, maximiert. Folglich ist der Umschlingungswinkel des Federbügels um das eingesetzte Eindrehinstrument herum in Anzugsrichtung maximiert. Die Durchbrechung ist derart ausgebildet, dass die Rastnase sich radial nach aussen bezüglich der Rotationsachse wenigstens um die Arbeitslänge bewegen kann, sodass sie ihre Freigabestellung erreichen kann.

In einer bevorzugten Ausführungsform ist die Durchbrechung durch einen wenigstens annähernd radial verlaufenden Schnitt ausgebildet. Somit kann die Durchbrechung einfach ausgebildet werden, insbesondere bei einer Umfassung mit einer dünnen Wandung.

In einer bevorzugten Ausführungsform ist die Durchbrechung durch einen wenigstens annähernd axial verlaufenden Schnitt ausgebildet. Bei einer Umfassung mit einer dickeren Wandung als in der vorherigen Ausführungsform kann die Durchbrechung ebenfalls einfach ausgebildet werden.

In einer bevorzugten Ausführungsform kann die Durchbrechung derart ausgebildet sein, dass die Bewegung des Federbügels in Umfangsrichtung begrenzt ist.

Der Federbügel weist auf seinem freien Ende eine durch die Durchbrechung definierte, wenigstens annähernd radial zur Rotationsachse verlaufende Stirnfläche auf, welche eine durch die Durchbrechung definierte, wenigstens annähernd radial zur Rotationsachse verlaufende Gegenstirnfläche des Halsbereichs zugewandt ist.

In einer bevorzugten Ausführungsform ist die Breite der Durchbrechung, in Umfangsrichtung gemessen, derart bemessen, dass die Stirnfläche des Federbügels bei einer Verformung durch eine Kompression mindestens teilweise in Anlage mit der Gegenstirnfläche des Halsbereichs kommen kann, wenn die Verformung des Federbügels ein vorbestimmtes Mass erreicht hat. Die Breite der Durchbrechung wird derart bestimmt, dass die Verformung des Federbügels in dessen elastischen Bereich bleibt. Somit kann eine weitere Verformung des Federbügels und folglich eine Beschädigung des Drehmomentschlüssels verhindert werden.

Erfindungsgemäss bildet die Durchbrechung einen vom Halsbereich abstehenden, ersten Haken und einen vom Federbügel abstehenden, zweiten Haken. Der erste und der zweite Haken weisen einen ersten und einen zweiten Vorsprung auf, welche quer, vorzugsweise rechtwinklig zur Anzugsrichtung verlaufen und sich überlappen. Im Ruhezustand und beim normalen Betrieb des Drehmomentschlüssels sind der erste und der zweite Vorsprung voneinander beabstandet. Das heisst, dass der erste und der zweite Vorsprung so ausgebildet sind, dass ein Spalt zwischen letzteren im Ruhezustand und im normalen Betrieb vorhanden ist.

Um eine Überdehnung des Federbügels zu vermeiden kommen der erste und der zweite Vorsprung in Anlage, wenn die Verformung des Federbügels durch eine Ausdehnung ein vorbestimmtes Mass erreicht hat. Somit kann eine weitere Verformung des Federbügels und folglich eine Beschädigung des Drehmomentschlüssels verhindert werden. Eine Überdehnung kann beispielsweise beim Greifen und Herausnehmen des Drehmomentschlüssels aus einer Chirurgiekassette erfolgen, wenn zum Beispiel der Drehmomentschlüssel und andere Werkzeuge ineinandergreifen und am Drehmomentschlüssel zu fest gezogen wird.

Der Spalt wird derart bestimmt, dass die Verformung des Federbügels in dessen elastischen Bereich bleibt. Dies bedeutet, dass die Verformung reversible bleibt und der Federbügel seine ursprüngliche Form wieder aufnimmt, wenn keine Verformungskraft mehr aufgebracht wird. Somit kann sichergestellt werden, dass der Federbügel seine Eigenschaften beibehält, insbesondere die ausgeübte Kraft, gegen die die Rastnase in die Freigabestellung gelangen kann.

Wenn der Aussendurchmesser des Ratschenkopfs des eingesetzten Eindrehinstruments kleiner als der Innendurchmesser der Aufnahmeöffnung ist, kann der Spalt im normalen Betrieb kleiner als im Ruhezustand sein.

Die durch den wenigstens annähernd radial verlaufenden Schnitt ausgebildete Durchbrechung kann, in Längsschnitt der Umfassung gesehen, wenigstens annähernd S-förmig und von der Vorderseite zur Rückseite des Drehmomentschlüssels verlaufen, wobei eine der Vorderseite zugewandte erste Hälfte der S-Form den ersten Haken und eine der Rückseite zugewandte zweite Hälfte der S-Form den zweiten Haken bilden.

Möglich ist ebenfalls, dass die durch den wenigstens annähernd axial verlaufenden Schnitt ausgebildete Durchbrechung, im Querschnitt der Umfassung gesehen, wenigstens annähernd S-förmig und von einer der Rotationsachse zugewandten Innenseite der Umfassung zu einer der Rotationsachse abgewandten Aussenseite der Umfassung verläuft. In dieser Ausführungsform bildet die der Innenseite zugewandte erste Hälfte der S-Form den ersten Haken und eine der Aussenseite zugewandte erste Hälfte der S-Form bildet den zweiten Haken.

In einer bevorzugten Ausführungsform mündet die Durchbrechung in eine im Halsbereich ausgebildete Aussparung, welche den ersten Haken aufweist. Ferner ragt der vom Federbügel abstehende, zweite Haken in die Aussparung hinein, um mit dem ersten Haken in Eingriff zu kommen, wenn die Verformung des Federbügels durch eine Kompression oder eine Ausdehnung ein vorbestimmtes Mass erreicht hat. Dadurch dass der Eingriff des ersten und des zweiten Hakens eine weitere Bewegung des Federbügels bezüglich des Halsbereichs verhindern, kann eine weitere Verformung des Federbügels und folglich eine Beschädigung des Drehmomentschlüssels verhindert werden.

In einer bevorzugten Ausführungsform des Drehmomentschlüssels ist der Federbügel auf der dem Betätigungshebel abgewandten Seite des Halsbereichs von diesem abstehend ausgebildet. Diese Anordnung ermöglicht eine einfache Handhabung des Drehmomentschlüssels beim Einsatz im Mund des Patienten.

Bevorzugt ist der Federbügel integral und einstückig mit dem Halsbereich ausgebildet. Dies vereinfacht die Reinigung, da die Abwesenheit einer Verbindungsstelle zwischen dem Halsbereich und dem Federbügel eine Schmutzablagerung an der Verbindungsstelle verhindert.

In einer bevorzugten Ausführungsform des Drehmomentschlüssels weist ein Abschnitt des Federbügels einen reduzierten Querschnitt auf, um die wenigstens annähernd in radialer Richtung vom Federbügel auf das eingesetzte Eindrehinstrument ausgeübte Kraft zu dimensionieren. Diese Konstruktion ist einfach in die Herstellung des Drehmomentschlüssels umsetzbar und bildet eine günstige Variante zum Erreichen der erwünschten Kraft des Federbügels. Die Reduktion des Querschnitts des Federbügels zielt darauf ab, die Kraft des Federbügels zu reduzieren, sodass das ans Eindrehinstrument durch Reibung übertragene Drehmoment in der Freigabestellung der Rastnase minimal ist. Dadurch kann ein unerwünschtes Losschrauben des Implantats oder des Verbindungselements in die Freigabestellung vermieden werden.

Der Abschnitt des Federbügels mit dem reduzierten Querschnitt kann durch eine Aussparung im Federbügel gebildet sein. Beispielsweise kann diese Aussparung durch einen radial zur Rotationsachse verlaufenden Einschnitt im Federbügel gebildet sein. Bei gleichem Material für den Federbügel ist es jedoch auch möglich, einen über die gesamte Länge des Federbügels konstanten jedoch dünneren Querschnitt des Federbügels zu wählen, um dieselbe ausgeübte Kraft zu erreichen. Die Bestimmung der Kraft des Federbügels ist in diesem Fall einfach, jedoch kann der dünne Querschnitt eine Schwachstelle für bestimmte Materialien bilden. Ferner ist es auch möglich die ausgeübte Kraft des Federbügels durch die Wahl eines passenden Materials zu dimensionieren. Je nach zu erfüllenden Bedingungen bei der Dimensionierung der Kraft des Federbügels kann der Fachmann eine der oben erwähnten Lösung wählen oder diese Lösungen kombinieren.

In einer bevorzugten Ausführungsform des Drehmomentschlüssels ist die Umfassung wenigstens annähernd kreisringförmig ausgebildet. Diese Form der Umfassung passt zu einer Mehrheit der Eindrehinstrumente, sodass der Drehmomentschlüssel breit eingesetzt werden kann. Ferner kann diese Form einfach und günstig hergestellt werden.

In einer besonders bevorzugten Ausführungsform des Drehmomentschlüssels mit einer wenigstens annähernd kreisringförmigen Umfassung, erstreckt sich eine Tangente zu der der Rotationsache zugewandten Umfangsfläche des Federbügels an einem Punkt des Abschnitts, an welchem, in radialer Richtung gemessen, der Federbügel den wenigstens annähernd kleinsten Querschnitt aufweist, wenigstens annähernd parallel zu einem Radius, welcher Radius von der Rotationsachse zum Mitnahmeabschnitt verläuft. Es ist jedoch auch möglich, die Tangente wenigstens annähernd parallel zu einer Normale bezüglich der Angriffsfläche, welche bevorzugt eine Ebene ist, zu gestalten. Diese Anordnungen bieten eine optimale Verteilung der Spannungen in der Umfassung, sodass das Risiko eines Bruchs oder einer nicht elastischen Verformung des Federbügels reduziert werden kann.

Bei der Dimensionierung der durch den Federbügel auf das Eindrehinstrument, bei der Drehung entgegen der Anzugsrichtung ausgeübten Kraft muss sichergestellt werden, dass das übertragene Drehmoment, d.h. das in der Freigabestellung der Rastnase übertragene Drehmoment, einen vorbestimmten Drehmomentgrenzwert nicht überschreitet, welcher zu einem Ausschrauben des Implantats bzw. des Verbindungselements führen könnte. Somit kann der Drehmomentschlüssel seine Ratschenfunktion bei einer Drehung entgegen der Anzugsrichtung erfüllen.

In einer bevorzugten Ausführungsform umfasst der Drehmomentschlüssel einen vom Halsbereich auf der dem Kopfbereich abgewandten Seite des Halsbereichs abstehenden, sich in einer wenigstens annähernd parallel zur Ebene erstreckenden Schaftbereich und einen vom freien Endbereich des Schaftbereichs abstehenden Indikatorbereich, wobei der Betätigungshebel sich mindestens bis zum Indikatorbereich erstreckt. Bevorzugt erstreckt sich der Betätigungshebel bis in den Indikatorbereich.

Vorzugsweise ist der Betätigungshebel einstückig und integral mit dem Halsbereich ausgebildet, um die Reinigung zu vereinfachen.

Besonders bevorzugt erstreckt sich der Betätigungshebel über den Indikatorbereich hinaus, um eine einfache Bedienung des Betätigungshebels über dessen dem Indikatorbereich hinausgehenden Abschnitt zu ermöglichen.

Eine Ruheposition bzw. eine Auslenkposition des Betätigungshebels definiert seine Lage, wenn keine bzw. eine Kraft auf den Betätigungshebel ausgeübt wird, d.h. wenn der Betätigungshebel keine bzw. eine Biegung aufweist.

Vorzugsweise ist der Schaftbereich, in Anzugsrichtung gesehen, abwärts des Betätigungshebels an dem Halsbereich befestigt und verläuft der Schaftbereich parallel zu dem sich in seiner Ruheposition befindenden Betätigungshebel. Diese Anordnung bietet eine kompakte Form des Drehmomentschlüssels, sodass der Drehmomentschlüssel wenig Platz in der Chirurgiekassette einnimmt. Ferner greifen der Drehmomentschlüssel und andere Werkzeuge somit in der Chirurgiekassette nicht ineinander.

Vorzugsweise ist ein Spalt zwischen dem Schaftbereich und dem Betätigungshebel vorhanden, um eine einfache Reinigung zu ermöglichen.

Vorzugsweise erstreckt sich der Indikatorbereich ebenfalls wenigstens annähernd parallel zur Ebene, beispielsweise in der Form eines Plättchens. Besonders bevorzugt ist der Indikatorbereich einstückig und integral mit dem Schaftbereich ausgebildet. Diese Ausführungsform ist konstruktiv besonders einfach und ihre Herstellung kostengünstig.

Es ist ebenfalls möglich den Schaftbereich mit einem ersten und einem zweiten Arm auszubilden, welche sich wenigstens annähernd parallel zur Ebene und vom Halsbereich auf der dem Kopfbereich abgewandten Seite erstrecken, wobei das freie Ende des ersten und des zweiten Arms über den Indikatorbereich verbunden sind und der Betätigungshebel an den Halsbereich zwischen dem ersten und dem zweiten Arm angeordnet ist. Der erste und zweite Arm bilden zusammen mit dem Indikatorbereich einen Griff, vorzugsweise in der Form eines spitzwinkligen Dreiecks, welcher eine einfache Handhabung des Drehmomentschlüssels erlauben, insbesondere bei dessen Aufsetzen auf das Eindrehinstrument.

Vorzugsweise ist der Schaftbereich biegefest, sodass die relative Bewegung des Betätigungshebels bezüglich des Schaftbereichs zuverlässig und einfach reproduziert werden kann.

In einer besonders bevorzugten Ausführungsform des Drehmomentschlüssels ist der Betätigungshebel biegbar, vorzugsweise elastisch biegbar, besonders bevorzugt linearelastisch biegbar. Mit einem elastisch biegbaren Betätigungshebel wird sichergestellt, dass die Biegung reversible bleibt und der Betätigungshebel seine ursprüngliche Form wieder aufnimmt, wenn keine Kraft mehr aufgebracht wird. Somit kann die Anzeigegenauigkeit des aufgebrachten Drehmoments über die Lebensdauer des Drehmomentschlüssels gewährleistet sein. Ein linearelastisch biegbarer Betätigungshebel weist eine proportional zur einwirkenden Kraft resultierende Biegung auf, welche eine zuverlässige und praktische Bedienung des Drehmomentschlüssels ermöglicht.

Bei der Aufbringung einer Kraft in Anzugsrichtung auf einen freien Endbereich des Betätigungshebels ist eine Auslenkung des Betätigungshebels ausgehend von dessen Ruheposition ein Mass für das generierte Drehmoment. Vorzugsweise umfasst der Betätigungshebel auf seinem dem Indikatorbereich überlappenden Abschnitt einen Nocken und umfasst der Indikatorbereich ein Langloch, welches dazu bestimmt ist, den Nocken aufzunehmen. Bei der Biegung des Betätigungshebels von seiner Ruheposition mindestens bis zu einer maximalen vorbestimmten Biegung bewegt sich der Nocken entlang des Langloches. Besonders bevorzugt ist das Langloch derart ausgebildet, dass es einen Endanschlag für den Nocken bildet, wenn die Biegung des Betätigungshebels dem maximalen aufzubringenden Drehmoment entspricht. Dadurch kann ein Überdehnen und somit eine Verformung ausserhalb des elastischen Bereichs des Betätigungshebels annähernd vollständig vermieden werden.

In einer noch bevorzugteren Ausführungsform des Drehmomentschlüssels sind auf dem Indikatorbereich Messmarkierungen angebracht, an welchen die Auslenkung des Betätigungshebels als erzeugtes Drehmoment ablesbar ist. Vorzugsweise sind die Messmarkierungen entlang des Langlochs angebracht, sodass das aufgebrachte Drehmoment über die Position des Nockens im Langloch bezüglich den Messmarkierungen abgelesen werden kann. Vorzugsweise ist der Nocken fluchtend mit der Oberfläche des Indikatorbereichs ausgebildet, sodass ein Parallaxenfehler beim Auswerten der Übereinstimmung des Nockens mit einer Messmarkierung vermieden werden kann.

Zur Herstellung des Drehmomentschlüssels sind Werkstoffe geeignet, welche bereits in der Medizinaltechnik eingesetzt werden und biokompatibel, reinigbar sowie sterilisierbar sind. Geeignete Materialien sind hierbei beispielsweise Metall und Metalllegierungen wie Stahl, rostfreier Stahl, Titan, Titanlegierungen und Kunststoff.

Der Drehmomentschlüssel kann auch zum Lösen des Implantats bzw. des Verbindungselements gebraucht werden, indem der Drehmomentschlüssel um den Betätigungshebel um 180° gedreht wird und auf das Eindrehinstrument wieder aufgesetzt wird.

Weitere Vorteile und Eigenschaften der Erfindung gehen aus der nachstehenden Beschreibung eines Ausführungsbeispiels hervor, welches anhand der beiliegenden Figuren erläutert wird.

Diese zeigen rein schematisch:
- Fig. 1: eine perspektivische Ansicht eines nichterfindungsgemässen Drehmomentschlüssels;
- Fig. 2: eine Draufsicht des Drehmomentschlüssels gemäss Fig. 1;
- Fig. 3: eine Ansicht der Rückseite des Drehmomentschlüssels gemäss Fig. 1;
- Fig. 4: eine Seitenansicht eines herkömmlichen Eindrehinstruments;
- Fig. 5: eine Draufsicht des Kopfbereichs mit dem im Querschnitt dargestellten, eingesetzten Eindrehinstrument der Fig. 4;
- Fig. 6: eine perspektivische Ansicht des Kopf- und Halsbereichs einer erfindungsgemässen Ausführungsform des Drehmomentschlüssels;
- Fig. 7: eine Draufsicht des Kopf- und Halsbereichs einer weiteren erfindungsgemässen Ausführungsform des Drehmomentschlüssels; und
- Fig. 8: eine Draufsicht des Kopf- und Halsbereichs einer weiteren erfindungsgemässen Ausführungsform des Drehmomentschlüssels.

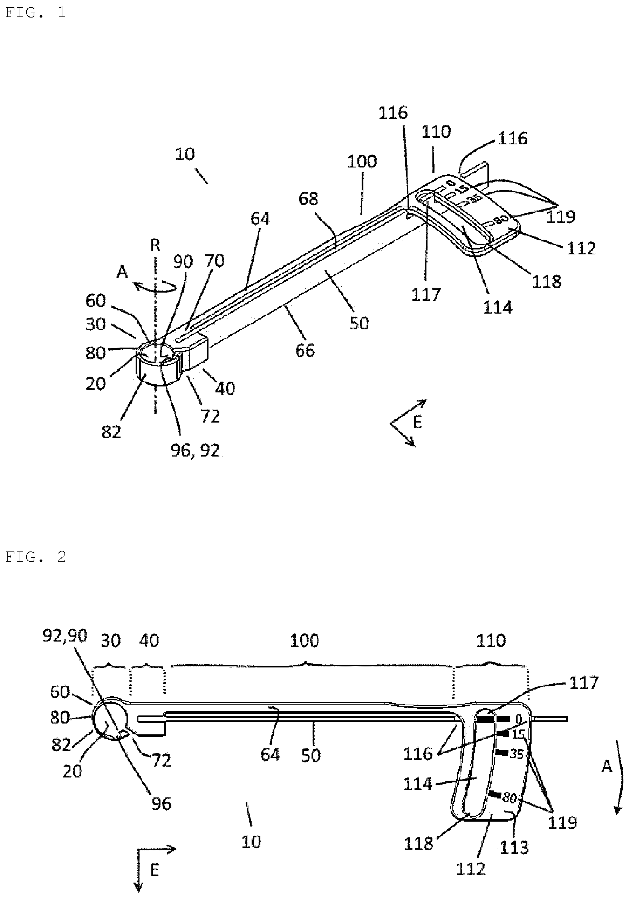

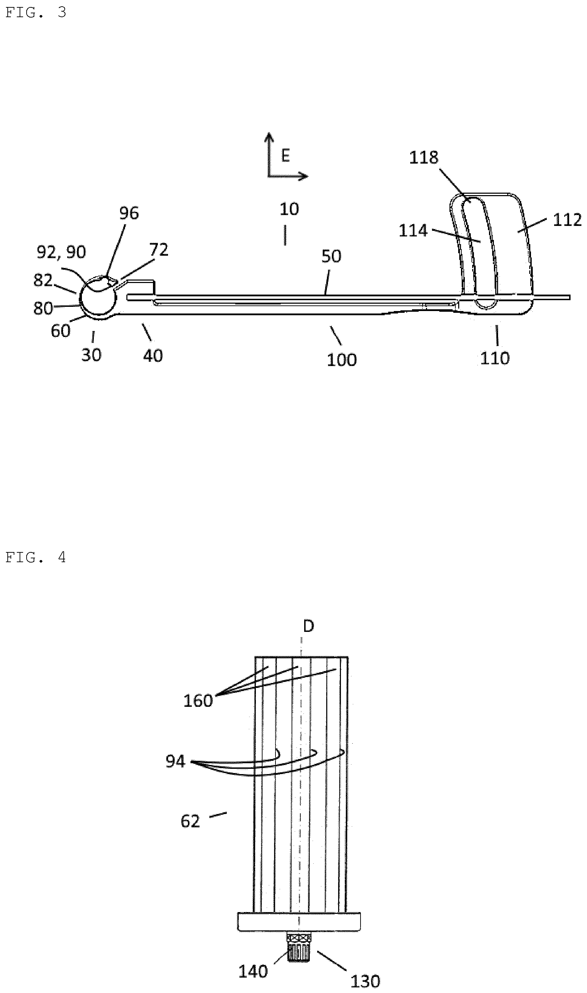

Der in der Figurenfolge 1, 2 und 3 abgebildete Drehmomentschlüssel 10 umfasst einen eine Aufnahmeöffnung 20 aufweisenden Kopfbereich 30, einen an den Kopfbereich 30 anschliessenden Halsbereich 40, und einen am Halsbereich 40 befestigten, stabförmigen, im Querschnitt rechteckförmigen, biegbaren Betätigungshebel 50 zum Aufbringen eines Drehmoments auf den Kopfbereich 30. Die Aufnahmeöffnung 20 des Kopfbereichs 30 ist durch eine kreisringförmige Umfassung 60 gebildet, welche eine Rotationsachse R definiert. Die Umfassung 60 ist dazu bestimmt, ein Eindrehinstrument 62 in der Erstreckung der Rotationsachse R aufzunehmen, welches in Fig. 4 näher beschrieben wird. Eine Ebene E erstreckt sich rechtwinklig zur Rotationsachse R und umfasst eine Vorderseite 64 des Drehmomentschlüssels 10, welche Vorderseite 64 in Fig. 2 dargestellt ist. Eine Rückseite 66 des Drehmomentschlüssels 10 ist in Fig. 3 abgebildet.

Eine Vorderseite 68 des Betätigungshebels 50 erstreckt sich in der Ebene E. Ferner ist die Ausdehnung des Querschnitts des Betätigungshebels 50 grösser in Richtung rechtwinklig zur Ebene E als in Richtung parallel zur Ebene E.

Der Halsbereich 40 bildet ein wenigstens annähernd quaderförmiges, massives Teil und umfasst einen sich rechtwinklig zur Ebene E verlaufenden Einschnitt 70 zur Aufnahme eines Endbereichs des Betätigungshebels 50 und dessen Befestigung.

Weiter weist die Umfassung 60 eine über ihren gesamten Querschnitt verlaufende Durchbrechung 72 auf und bildet die Umfassung 60 einen Federbügel 80. Der Federbügel 80 ist auf der dem Betätigungshebel 50 abgewandten Seite des Halsbereichs 40 von diesem abstehend, integral und einstückig mit dem Halsbereich 40 ausgebildet. Die Durchbrechung 72 grenzt an den Halsbereich 40 an und ist durch einen sich wenigstens annähernd radial zur Rotationsachse R erstreckenden Schlitz 72 gebildet.

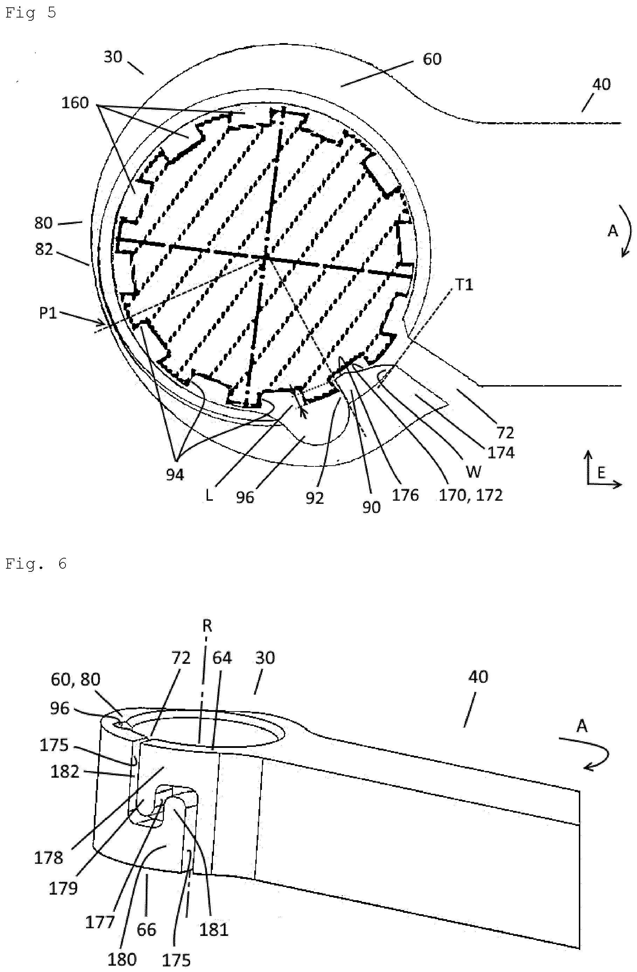

Ein Abschnitt 82 des Federbügels 80 weist einen reduzierten Querschnitt auf, welcher in der Fig. 5 näher beschrieben wird.

Eine Rastnase 90 ist an dem Federbügel 80, integral und einstückig mit dem Federbügel 80, an die Durchbrechung angrenzend ausgebildet. In ihrer Ruhestellung ragt die Rastnase 90 in die Aufnahmeöffnung 20 hinein. Die Ruhestellung ist die Position der Rastnase 90, wenn kein Eindrehinstrument in die Aufnahmeöffnung 20 eingesetzt ist und keine Kraft in radialer Richtung auf den Federbügel 80 ausgeübt wird.

Ferner weist die Rastnase 90 einen Mitnahmeabschnitt 92 auf, welcher dazu bestimmt ist, bei in die Aufnahmeöffnung 20 eingesetztem Eindrehinstrument 62, bei einer Drehung in Anzugsrichtung A des Betätigungshebels 50 in einer Mitnahmestellung der Rastnase 90 mit einer Gegenfläche 94 des Eindrehinstruments 62, welches auf Fig. 4 und 5 ersichtlich ist, zusammenzuwirken. Beim Zusammenwirken des Mitnahmeabschnitts 92 mit der Gegenfläche 62 wird ein Drehmoment durch eine formschlüssige Mitnahme ans Eindrehinstrument 62 übertragen.

Die Rastnase 90 ist dazu bestimmt, bei einer Drehung entgegen der Anzugsrichtung A, gegen die Kraft des Federbügels 80 sich in radialer Richtung gegen aussen in eine Freigabestellung zu bewegen, um einen Freilauf zwischen dem Kopfbereich 30 und dem Eindrehinstrument 62 zu bilden.

Zudem weist der Federbügel 80 auf seiner der Rotationsachse R zugewandten Seite eine, in Anzugsrichtung A gesehen, bezüglich der Mitnahmefläche 92 vorlaufende, an die Mitnahmefläche 92 anschliessende, halbkreisförmige Ausnehmung 96 auf.

Der Drehmomentschlüssel 10 umfasst ebenfalls einen vom Halsbereich 40 auf der dem Kopfbereich 30 abgewandten Seite des Halsbereichs 40 abstehenden, sich in einer wenigstens annähernd parallel zur Ebene E erstreckenden, biegefesten Schaftbereich 100 und einen vom freien Endbereich des Schaftbereichs 100 abstehenden Indikatorbereich 110, wobei der Betätigungshebel 50 sich über den Indikatorbereich 110 hinaus erstreckt.

Der Schaftbereich 100 ist, in Anzugsrichtung A gesehen, abwärts des Betätigungshebels 50 an dem Halsbereich 40 befestigt, einstückig und integral mit dem Halsbereich 40 ausgebildet und verläuft parallel zu dem sich in seiner Ruheposition befindenden Betätigungshebel 50, wobei ein Spalt zwischen dem Schaftbereich 100 und dem Betätigungshebel 50 vorhanden ist.

Der Indikatorbereich 110 erstreckt sich in der Form eines Plättchens 112, welches eine in der Ebene E verlaufende Vorderfläche 113 aufweist, und ist einstückig und integral mit dem Schaftbereich 100 ausgebildet.

Der Betätigungshebel 50 umfasst auf seinem dem Indikatorbereich 110 überlappenden Abschnitt eine Aussparung 116, welche einen Freiraum 116 für den Indikatorbereich 110 bildet. Der Freiraum 116 ist derart ausgebildet, dass er die Bewegung der Vorderseite 68 des Betätigungshebels 50 in der Ebene E, d.h. in derselben Ebene wie die Vorderfläche 113 des Plättchens 112, bei Betätigung des Betätigungshebels 50 erlaubt. Die Aussparung 116 ist durch zwei rechteckförmige Einschnitte gebildet, deren Höhe, in Richtung rechtwinklig zur Ebene E gemessen, der Dicke des Plättchens 112 wenigstens annähernd entspricht, und welche von einem in der Ebene E verlaufenden Abschnitt des Betätigungshebels 50 getrennt sind. Der Abschnitt des Betätigungshebels 50 bildet einen Nocken 117, welcher als Zeiger 117 dient und sich in ein im Plättchen 112 ausgebildetes Langloch 114 bei Betätigung des Betätigungshebels 50 bewegt. Der Nocken 117 ist somit fluchtend mit der Vorderfläche 113 des Indikatorbereichs 110 ausgebildet. Das Langloch 114 erstreckt sich in der Form eines Kreisbogens, dessen Mittelpunkt über den Krümmungsmittelpunkt des betätigten Betätigungshebels 50 definiert ist.

Bei der Biegung des Betätigungshebels von seiner Ruheposition mindestens bis zu einer maximalen vorbestimmten Biegung bewegt sich der Zeiger 117 entlang des Langloches 114. Das Langloch 114 ist derart ausgebildet, dass es einen Endanschlag 118 für den Nocken 117 bildet, wenn die Biegung des Betätigungshebels 50 dem maximalen aufzubringenden Drehmoment entspricht.

Auf dem Indikatorbereich 110 sind Messmarkierungen 119 entlang des Langlochs 114 angebracht, an welchen die Auslenkung des Betätigungshebels 50 als erzeugtes Drehmoment ablesbar ist. Bei der Aufbringung einer Kraft in Anzugsrichtung A auf einen freien Endbereich des Betätigungshebels 50 ist eine Auslenkung des Betätigungshebels 50 ausgehend von dessen Ruheposition ein Mass für das generierte Drehmoment.

Je nach Zahnimplantatsystem sind Drehmomente im Bereich von 10 Ncm bis 100 Ncm typischerweise erforderlich.

Fig. 4 zeigt ein herkömmliches Eindrehinstrument 62 umfassend einen zylindrischen, eine Drehachse D des Eindrehinstruments definierenden Ratschenkopf 120, welcher in die Aufnahmeöffnung 20 des Drehmomentschlüssels 10 eingesetzt werden kann, und einen an den Ratschenkopf 120 anschliessenden, sich in Richtung der Drehachse D erstreckenden Schaft 130. Das freie Ende 140 des Schaftes weist ein standardisiertes Profil zum Zusammenwirken mit einem Implantat bzw. einem Verbindungselement auf, sodass das Eindrehinstrument 62 das auf den Ratschenkopf 120 aufgebrachte Drehmoment an das Implantat bzw. das Verbindungselement übertragen kann. Der Ratschenkopf 120 weist eine Umfangsfläche 150 auf, welche eine die Gegenfläche 94 bildende Konturierung umfasst, um eine Übertragung des auf den Drehmomentschlüssel 10 aufgebrachten Drehmoments an das Eindrehinstrument 62 zu ermöglichen. Die Konturierung umfasst eine Mehrzahl von parallel zur Drehachse D des Eindrehinstruments 62 verlaufenden Nuten 160, welche gleich ausgebildet und, in Umfangsrichtung gesehen, im gleichen Abstand zueinander angeordnet sind. Die Nuten 160 weisen einen mindestens annähernd rechteckigen Querschnitt auf, deren in Anzugsrichtung bezüglich der Mitnahmefläche vorlaufende Wand 94 bevorzugt wenigstens annähernd radial in Richtung zur Drehachse D verläuft und die Gegenfläche 94 bildet.

In Fig. 5 ist die Zusammenwirkung des Mitnahmeabschnitts 92 der Rastnase 90 mit der Gegenfläche 94 des eingesetzten Eindrehinstruments 62 in Anzugsrichtung A dargestellt. Der Mitnahmeabschnitt 92 ist durch die wenigstens annähernd radial zur Rotationsachse R verlaufende Mitnahmefläche 92 gebildet, welche dazu bestimmt, mit der wenigstens annähernd radial zur Rotationsachse R verlaufenden Gegenfläche 94 des eingesetzten Eindrehinstruments 62 zusammenzuwirken.

Die radial zur Rotationsachse R gemessene Länge der Mitnahmefläche 92, welche in Kontakt mit der Gegenfläche 94 des Eindrehinstruments 62 kommt, bildet die Arbeitslänge L. Die Arbeitslänge L entspricht der minimalen Länge der benötigten Bewegung der Rastnase 90, bezüglich der Rotationsachse R radial nach aussen, sodass die Rastnase 90 ihre Freigabestellung erreichen kann.

Weiter weist die Rastnase 90 eine, in Anzugsrichtung A gesehen, der Mitnahmefläche 92 nachlaufende, an die Mitnahmefläche 92 anschliessende Angriffsfläche 170 auf, welche die Bewegung der Rastnase 90 in die Freigabestellung erlaubt.

Die Angriffsfläche 170 ist durch eine Abschrägung 172 gebildet, deren Abstand zur Rotationsachse R entgegen der Anzugsrichtung A ohne Unterbrechung zunimmt und welche den Freilauf erlaubt. Die Abschrägung 172 erstreckt sich mindestens annähernd bis zum freien Ende 174 des Federbügels 80.

Zudem umfasst die Angriffsfläche 170 eine in Umfangsrichtung verlaufende Verlängerungsfläche 176, welche an die Mitnahmefläche 92 direkt anschliesst und an welche die Abschrägung 172 direkt anschliesst.

Die Rastnase 90 ist so ausgebildet, dass, in Umfangsrichtung der Umfassung 60 gemessen, die Gesamtlänge der Abschrägung 172 zusammen mit der Verlängerungsfläche 176 länger als die Breite der Nuten 160 ist.

Ein in der Ebene E gemessener Winkel W zwischen der Abschrägung 172 und einer an dem, in Anzugsrichtung gesehen, nachlaufenden Endpunkt der Abschrägung verlaufenden Tangente T1 zur Umfangsrichtung beträgt in der dargestellten Ausführungsform ca. 30°.

Der Federbügel 80 weist an einem Punkt P1, in radialer Richtung gemessen, den wenigstens annähernd kleinsten Querschnitt auf. Der Punkt P1 befindet sich gegenüber der Angriffsfläche 170 wenigstens annähernd um 90° in Anzugsrichtung versetzt. Der Abschnitt 82 des Federbügels 80 mit dem reduzierten Querschnitt erstreckt sich beiderseits des Punktes P1 in der dargestellten Ausführungsform.

In Fig. 6 ist eine weitere Ausführungsform der Durchbrechung 72 dargestellt, welche durch einen radial verlaufenden Schnitt ausgebildet ist. Die Umfassung 60 weist eine dünne Wandung auf, d.h. einen Längsschnitt, welcher wesentlich grösser in axialer Richtung als in radialer Richtung ist.

Die Durchbrechung 72 ist derart ausgebildet, dass sie die Bewegung des Federbügels 80 gegenüber dem Halsbereich 40 in Umfangsrichtung in Anzugsrichtung A und entgegen der Anzugsrichtung A begrenzt ist.

Der Federbügel 80 weist auf seinem freien Ende 174 eine durch die Durchbrechung 72 definierte, wenigstens annähernd radial zur Rotationsachse R verlaufende Stirnfläche 175 auf, welche eine durch die Durchbrechung 72 definierte, wenigstens annähernd radial zur Rotationsachse verlaufende Gegenstirnfläche 177 des Halsbereichs 40 zugewandt ist.

Die Durchbrechung 72 und somit die Stirnfläche 175 und der Gegenstirnfläche 177 verlaufen, im Längsschnitt der Umfassung 60 gesehen, wenigstens annähernd S-förmig und von der Vorderseite 64 zur Rückseite 66 des Drehmomentschlüssels 10, wobei eine der Vorderseite 64 zugewandte erste Hälfte der S-Form einen vom Halsbereich 40 abstehenden, ersten Haken 178 und eine der Rückseite 66 zugewandte zweite Hälfte der S-Form den vom Federbügel 80 abstehenden, zweiten Haken 180 bilden.

Ein Spalt 182 zwischen dem ersten und dem zweiten Haken 178 bzw. 180 ist im Ruhezustand und im normalen Betrieb des Drehmomentschlüssels vorhanden. Der erste und der zweite Haken 178 bzw. 180 weisen einen ersten bzw. zweiten Vorsprung 179 bzw. 181 auf, welche in Anlage kommen, wenn die Verformung des Federbügels 80 ein vorbestimmtes Mass erreicht hat, und somit eine weitere Verformung des Federbügels 80 verhindern. Hierzu sei noch erwähnt, dass die Verformung in Anzugsrichtung sowie entgegen der Anzugsrichtung verhindert werden kann.

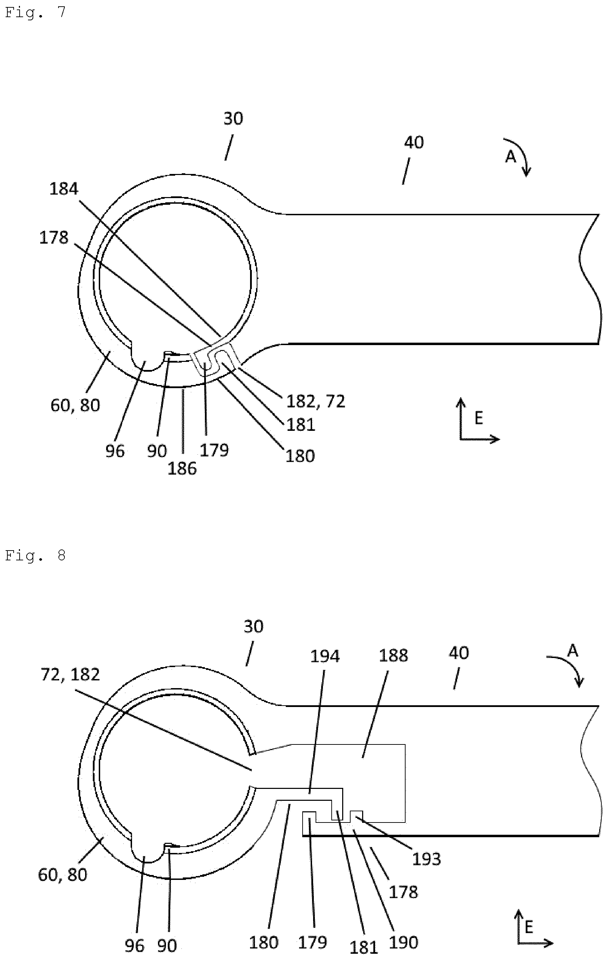

In Fig. 7 ist eine weitere Ausführungsform der Durchbrechung 72 dargestellt, welche durch einen axial verlaufenden Schnitt ausgebildet ist. Im Gegensatz zur Ausführungsform der Fig. 6 weist die Umfassung 60 eine dicke Wandung auf.

Die Durchbrechung 72 verläuft, im Querschnitt der Umfassung 60 gesehen, d.h. in einer parallel zur Ebene E verlaufenden Ebene, wenigstens annähernd S-förmig und von einer der Rotationsachse zugewandten Innenseite 184 der Umfassung 60 zu einer der Rotationsachse abgewandten Aussenseite 186 der Umfassung 60. Die der Innenseite 184 zugewandte erste Hälfte der S-Form bildet den vom Halsbereich 40 abstehenden, ersten Haken 178 und die der Aussenseite 186 zugewandte zweite Hälfte der S-Form den vom Federbügel 80 abstehenden, zweiten Haken 180.

Bei einer Verformung des Federbügels 80 ist die Zusammenwirkung des ersten und des zweiten Hakens 178 bzw. 180 und deren ersten und zweiten Vorsprung 179 bzw. 181 gleich wie bei der Ausführungsform der Fig. 6.

In Fig. 8 ist eine weitere Ausführungsform der Durchbrechung 72 dargestellt, bei welcher die Durchbrechung 72 in eine im Halsbereich 40 ausgebildete, mit der Aufnahmeöffnung 20 verbundene, rechteckige Aussparung 188 mündet. Die Aussparung 188 weist eine wenigstens annähernd radial zur Rotationsachse R gerichtete, in Anzugsrichtung A gesehen, der Durchbrechung 72 nachlaufende Wand 190 auf, von welcher der zweite und ein weiterer Vorsprung 179 bzw. 193 in Richtung auf das Innere der Aussparung 188 abstehen.

Der zweite Haken 180 umfasst einen, bezüglich der Rotationsachse R, radial nach aussen vom Federbügel 80 abstehenden Arm 194, welcher in die Aussparung 188 hineinragt und zwischen dem zweiten und dem weiteren Vorsprung 179 bzw. 193 liegt. Das freie Ende des Armes 194 weist den in Richtung gegen die Wand 190 gerichteten, ersten Vorsprung 181 auf. Der erste Vorsprung 181 kommt in Anlage mit dem zweiten Vorsprung 179, um die Bewegung des Federbügels 80 durch eine Ausdehnung zu begrenzen, wenn die Verformung des Federbügels 80 ein vorbestimmtes Mass erreicht hat. Somit kann eine Überdehnung des Federbügels 80 verhindern werden Hingegen kommt der erste Vorsprung 181 in Anlage mit dem weiteren Vorsprung 193, um die Bewegung des Federbügels 80 durch eine Kompression zu begrenzen..

Der Vollständigkeit halber sei noch erwähnt, dass in einer ähnlichen Ausführungsform wie die Ausführungsform der Fig. 8 der erste Haken 178 einen Vorsprung und der zweite Haken 180 zwei Vorsprünge aufweisen können. Mutatis mutandis ist die Zusammenwirkung des ersten und des zweiten Hakens 178 bzw. 180 und deren jeweiligen Vorsprünge bei einer Verformung des Federbügels 80 gleich wie bei der Ausführungsform der Fig. 8.

**Bezugszeichen**

| | |
|---|---|
| Ebene | E |
| Rotationsachse | R |
| Arbeitslänge | L |
| Anzugsrichtung | A |
| Drehrichtung | D |
| Tangente | T1 |
| Punkt | P1 |
| Winkel | W |
| Drehmomentschlüssel | 10 |
| Aufnahmeöffnung | 20 |
| Kopfbereich | 30 |
| Halsbereich | 40 |
| Betätigungshebel | 50 |
| Umfassung | 60 |
| Eindrehinstrument | 62 |
| Vorderseite | 64 |
| Rückseite | 66 |
| Vorderseite | 68 (des Betätigungshebels) |
| Einschnitt | 70 |
| Durchbrechung, Schlitz | 72 |
| Federbügel | 80 |
| Abschnitt des Federbügels | 82 |
| Rastnase | 90 |
| Mitnahmeabschnitt, Mitnahmefläche der Rastnase | 92 |
| Gegenfläche des Eindrehinstruments | 94 |
| Ausnehmung | 96 |
| Schaftbereich | 100 |
| Indikatorbereich | 110 |
| Plättchen | 112 |
| Vorderfläche | 113 |
| Langloch | 114 |
| Aussparung, Freiraum | 116 |
| Nocken, Zeiger | 117 |
| Endanschlag | 118 |
| Messmarkierungen | 119 |
| Ratschenkopf | 120 |
| Schaft | 130 |
| freies Ende des Schafts | 140 |
| Umfangsfläche | 150 |
| Nuten | 160 |
| Angriffsfläche | 170 |
| Abschrägung | 172 |
| freies Ende des Federbügels | 174 |
| Stirnfläche | 175 |
| Verlängerungsfläche | 176 |
| Gegenstirnfläche | 177 |
| erster Haken | 178 |
| erster Vorsprung | 179 |
| zweiter Haken | 180 |
| zweiter Vorsprung | 181 |
| Spalt | 182 |
| Innenseite der Umfassung | 184 |
| Aussenseite der Umfassung | 186 |
| Aussparung | 188 |
| Wand | 190 |
| Arm | 194 |
| weiterer Vorsprung | 193 |

## Patentansprüche

1. Drehmomentschlüssel (10) als Ratscheninstrument für die Medizinaltechnik, insbesondere die Dentalmedizin, mit einem eine Aufnahmeöffnung (20) aufweisenden Kopfbereich (30), einem an den Kopfbereich (30) anschliessenden Halsbereich (40), einem am Halsbereich (40) befestigten, sich wenigstens annähernd in einer Ebene (E) erstreckenden, stabförmigen Betätigungshebel (50) zum Aufbringen eines Drehmoments auf den Kopfbereich (30), einer die Aufnahmeöffnung (20) des Kopfbereichs (30) bildenden Umfassung (60), welche eine wenigstens annähernd rechtwinklig zur Ebene (E) verlaufende Rotationsachse (R) definiert und dazu bestimmt ist, ein Eindrehinstrument (62) in der Erstreckung der Rotationsachse (R) aufzunehmen, einem Federbügel (80), und einer an diesem ausgebildeten Rastnase (90), welche in ihrer Ruhestellung in die Aufnahmeöffnung (20) hineinragt und deren Mitnahmeabschnitt (92) dazu bestimmt ist, bei in die Aufnahmeöffnung (20) eingesetztem Eindrehinstrument (62), bei einer Drehung in Anzugsrichtung (D) des Betätigungshebels (50) in einer Mitnahmestellung der Rastnase (90) mit einer Gegenfläche (94) des Eindrehinstruments (62) zusammenzuwirken, um durch Mitnahme ein Drehmoment ans Eindrehinstrument (62) zu übertragen, und welche Rastnase (90) dazu bestimmt ist, bei einer Drehung entgegen der Anzugsrichtung gegen die Kraft des Federbügels (80) sich in eine Freigabestellung zu bewegen, um einen Freilauf zwischen dem Kopfbereich (30) und dem Eindrehinstrument (62) zu bilden,
wobei die Umfassung (60) eine über ihren gesamten Querschnitt verlaufende Durchbrechung (72) aufweist und den Federbügel (80) bildet,
wobei
die Durchbrechung (72) einen vom Halsbereich (40) abstehenden, ersten Haken (178) und einen vom Federbügel (80) abstehenden, zweiten Haken (180) bildet, und der erste und der zweite Haken (178) bzw. (180) einen ersten bzw. einen zweiten Vorsprung (179) bzw. (181) aufweisen, welche sich überlappen und derart ausgebildet sind, dass der erste und der zweite Vorsprung (179) bzw. (181) in Anlage kommen, wenn die Verformung des Federbügels (80) durch eine Ausdehnung ein vorbestimmtes Mass erreicht hat.

2. Drehmomentschlüssel (10) nach Anspruch 1, **dadurch gekennzeichnet, dass** die Rastnase (90) integral und einstückig mit dem Federbügel (80) ausgebildet ist.

3. Drehmomentschlüssel (10) nach Anspruch 1 oder 2, **dadurch gekennzeichnet, dass** die Rastnase (90) an die Durchbrechung (72) angrenzt.

4. Drehmomentschlüssel (10) nach einem der Ansprüche 1 bis 3, **dadurch gekennzeichnet, dass** der Mitnahmeabschnitt (92) durch eine wenigstens annähernd radial zur Rotationsachse (R) verlaufende Mitnahmefläche (92) gebildet ist, welche dazu bestimmt ist, mit der vorzugsweise wenigstens annähernd radial zur Rotationsachse (R) verlaufenden Gegenfläche (94) des eingesetzten Eindrehinstruments (62) zusammenzuwirken.

5. Drehmomentschlüssel (10) nach einem der Ansprüche 1 bis 4, **dadurch gekennzeichnet, dass** die Rastnase (90) eine, in Anzugsrichtung (D) gesehen, der Mitnahmefläche (92) nachlaufende, an die Mitnahmefläche (92) anschliessende Abschrägung (172) aufweist, deren Abstand zur Rotationsachse (R) entgegen der Anzugsrichtung (D) zunimmt und welche den Freilauf erlaubt.

6. Drehmomentschlüssel (10) nach einem der Ansprüche 1 bis 5, **dadurch gekennzeichnet, dass** der Federbügel (80) auf seiner der Rotationsachse (R) zugewandten Seite eine, in Anzugsrichtung D gesehen, bezüglich der Mitnahmefläche (92) vorlaufende, an die Mitnahmefläche (92) anschliessende Ausnehmung (96) aufweist.

7. Drehmomentschlüssel (10) nach einem der Ansprüche 1 bis 6, **dadurch gekennzeichnet, dass** die vorzugsweise durch einen sich wenigstens annähernd radial zur Rotationsachse (R) erstreckenden Schlitz (72) gebildete Durchbrechung (72) an den Halsbereich (30) angrenzt.

8. Drehmomentschlüssel (10) nach einem der Ansprüche 1 bis 7, **dadurch gekennzeichnet, dass** der Federbügel (80) auf der dem Betätigungshebel (50) abgewandten Seite des Halsbereichs (30) von diesem abstehend und bevorzugt integral und einstückig mit dem Halsbereich (30) ausgebildet ist.

9. Drehmomentschlüssel (10) nach einem der Ansprüche 1 bis 8, **dadurch gekennzeichnet, dass** ein Abschnitt (82) des Federbügels (80) einen reduzierten Querschnitt aufweist, um die wenigstens annähernd in radialer Richtung vom Federbügel (80) auf das eingesetzte Eindrehinstrument (62) ausgeübte Kraft zu dimensionieren.

10. Drehmomentschlüssel (10) nach einem der Ansprüche 1 bis 9, **dadurch gekennzeichnet, dass** die Umfassung (60) wenigstens annähernd kreisringförmig ausgebildet ist.

11. Drehmomentschlüssel (10) nach Anspruch 9 mit einer wenigstens annähernd kreisringförmigen Umfassung (60), **dadurch gekennzeichnet, dass** eine Tangente zu der der Rotationsache (R) zugewandten Umfangsfläche des Federbügels (80) an einem Punkt (P1) des Abschnitts (82), an welchem, in radialer Richtung gemessen, der Federbügel (80) den wenigstens annähernd kleinsten Querschnitt aufweist, sich wenigstens annähernd parallel zu einem Radius erstreckt, welcher von der Rotationsachse (R) zum Mitnahmeabschnitt (92) verläuft.

12. Drehmomentschlüssel (10) nach einem der Ansprüche 1 bis 11, **gekennzeichnet durch** einen vom Halsbereich (40) auf dessen dem Kopfbereich (30) abgewandten Seite abstehenden, sich in einer wenigstens annähernd parallel zur Ebene (E) erstreckenden Schaftbereich (100) und einen vom freien Endbereich des Schaftbereichs (100) abstehenden Indikatorbereich (110), wobei der Betätigungshebel (50) sich mindestens bis zum Indikatorbereich (110), bevorzugt bis in den Indikatorbereich (110) hinein, besonders bevorzugt über diesen hinaus erstreckt.

13. Drehmomentschlüssel (10) nach Anspruch 12, **dadurch gekennzeichnet, dass** der Betätigungshebel (50) biegbar, vorzugsweise linearelastisch biegbar ist, und dass bei der Aufbringung einer Kraft in Anzugsrichtung (D) auf einen freien Endbereich des Betätigungshebels (50) eine Auslenkung des Betätigungshebels (50) ausgehend von dessen Ruheposition ein Mass für das generierte Drehmoment ist.

14. Drehmomentschlüssel (10) nach Anspruch 13, **dadurch gekennzeichnet, dass** auf dem Indikatorbereich (110) Messmarkierungen (119) angebracht sind, an welchen die Auslenkung des Betätigungshebels (50) als erzeugtes Drehmoment ablesbar ist.

## Claims

1. A torque wrench (10) as a ratchet instrument for medical technology, in particular dentistry, having a head region (30) which has a receptacle opening (20), a neck region (40) which adjoins the head region (30), a bar-shaped activation lever (50) which, for applying a torque to the head region (30), is fastened to the neck region (40) and extends at least approximately in one plane (E), a bezel (60) which forms the receptacle opening (20) of the head region (30) and defines a rotation axis (R) that runs so as to be at least approximately orthogonal to the plane (E) and is specified for receiving a driver instrument (62) in the extent of the rotation axis (R), a spring bracket (80) and a latching cam (90) which is configured on said spring bracket (80) and in the resting position of said latching cam (90) protrudes into the receptacle opening (20), the entrainment portion (92) of said latching cam (20), when the driver instrument (62) is inserted in the receptacle opening (20), and when the activation lever (50) is rotated in the tightening direction (D) in an entraining position of the latching cam (90), being specified for interacting with a mating face (94) of the driver instrument (62) so as to by way of entrainment transmit a torque to the driver instrument (62), said latching cam (90), when rotating counter to the tightening direction, being specified for moving counter to the force of the spring bracket (80) to a releasing position, so as to form a freewheeling mechanism between the head region (30) and the driver instrument (62), wherein the bezel (60) has a cutout (72) running across the entire cross section of the latter and forms the spring bracket (80), wherein the bezel (60) is configured so as to be at least approximately annular, wherein the cutout (72) forms a first hook (178), which projects from the neck region (40), and a second hook (180), which projects from the spring bracket (80), and the first hook (178) and the second hook (180) have a first protrusion (179) and a second protrusion (181), respectively, which mutually overlap and are configured in such a manner that the first protrusion (179) and the second protrusion (181) come to bear on one another once the deformation of the spring bracket (80) as a result of expansion has reached a predetermined measure.

2. The torque wrench (10) as claimed in claim 1, **characterized in that** the latching cam (90) is configured so as to be integral to and in one piece with the spring bracket (80).

3. The torque wrench (10) as claimed in claim 1 or 2, **characterized in that** the latching cam (90) is adjacent to the cutout (72).

4. The torque wrench (10) as claimed in one of claims 1 to 3, **characterized in that** the entrainment portion (92) is formed by an entrainment face (92) which runs so as to be at least approximately radial to the rotation axis (R) and is specified for interacting with the mating face (94) of the inserted driver instrument (62) that preferably runs so as to be at least approximately radial to the rotation axis (R).

5. The torque wrench (10) as claimed in one of claims 1 to 4, **characterized in that** the latching cam (90) has a taper (172) which, when viewed in the tightening direction (D), trails the entrainment face (92) and adjoins the entrainment face (92), the spacing of said taper (172) from the rotation axis (R) increasing counter to the tightening direction (D) and permitting the freewheeling mechanism.

6. The torque wrench (10) as claimed in one of claims 1 to 5, **characterized in that** the spring bracket (80) on the side thereof that faces the rotation axis (R) has a clearance (96) which, when viewed in the tightening direction (D), leads the entrainment face (92) and adjoins the entrainment face (96).

7. The torque wrench (10) as claimed in one of claims 1 to 6, **characterized in that** the cutout (72) which is preferably formed by a slot (72) that extends so as to be at least approximately radial to the rotation axis (R) is adjacent to the neck region (30).

8. The torque wrench (10) as claimed in one of claims 1 to 7, **characterized in that** the spring bracket (80) on that side of the neck region (30) that faces away from the activation lever (50) projects from the latter and is preferably configured so as to be integral to and in one piece with the neck region (30).

9. The torque wrench (10) as claimed in one of claims 1 to 8, **characterized in that** a portion (82) of the spring bracket (80) has a reduced cross section so as to dimension the force which, by the spring bracket (80), is exerted at least approximately in the radial direction on the inserted driver instrument (62).

10. The torque wrench (10) as claimed in one of claims 1 to 9, **characterized in that** the bezel (60) is designed at least approximatively in the shape of a circular ring.

11. The torque wrench (10) as claimed in claim 9, **characterized in that** a tangent to that circumferential face of the spring bracket (80) that faces the rotation axis (R), at a point (P1) of the portion (82) where the spring bracket (80), when measured in the radial direction, has the at least approximately smallest cross section, extends so as to be at least approximately parallel to a radius, which radius runs from the rotation axis (R) to the entrainment portion (92).

12. The torque wrench (10) as claimed in one of claims 1 to 11, **characterized by** an indicator region (110) which, from the neck region (40), projects on that side of said neck region (40) that faces away from the head region (30), projects in a shaft region (100) that extends so as to be at least approximately parallel to the plane (E), and projects from the free end region of the shaft region (100), wherein the activation lever (50) extends at least up to the indicator region (110), preferably into the indicator region (110), particularly preferably beyond the latter.

13. The torque wrench (10) as claimed in claim 12, **characterized in that** the activation lever (50) is flexural, preferably flexural in a linear-elastic manner, and **in that**, when a force in the tightening direction (D) is applied to a free end region of the activation lever (50), a deflection of the activation lever (50), proceeding from the resting position of the latter, is a measure for the torque generated.

14. The torque wrench (10) as claimed in claim 13, **characterized in that** measurement marks (119) on which the deflection of the activation lever (50) can be read as the torque generated are attached to the indicator region (110).

## Revendications

1. Clé dynamométrique (10) en tant qu'instrument à cliquet pour la technique médicale, en particulier la dentisterie, comportant une zone de tête (30) avec une ouverture de réception (20), une zone de cou (40) adjacente à la zone de tête (30), un levier d'activation (50) en forme de barre qui, pour appliquer un couple à la zone de tête (30), est fixé à la zone de cou (40) et s'étend au moins approximativement dans un plan (E), une lunette (60) qui forme l'ouverture de réception (20) de la zone de tête (30) et définit un axe de rotation (R) qui s'étend de manière à être au moins approximativement orthogonal au plan (E) et est spécifiée pour recevoir un instrument de vissage (62) dans l'extension de l'axe de rotation (R), un étrier à ressort (80) et une came de verrouillage (90) qui est configurée sur ledit étrier à ressort (80) et dans la position de repos de ladite came de verrouillage (90) fait saillie dans l'ouverture de réception (20), la partie d'entraînement (92) de ladite came de verrouillage, lorsque l'instrument de vissage (62) est inséré dans l'ouverture de réception (20) et lorsque le levier d'activation (50) est tourné dans la direction de serrage (D) dans une position d'entraînement de la came de verrouillage (90), étant spécifiée pour interagir avec une face d'accouplement (94) de l'instrument de vissage (62) de manière à transmettre un couple à l'instrument de vissage (62) par le biais de l'entraînement, ladite came de verrouillage (90), lorsqu'elle tourne dans le sens inverse de la direction de serrage, étant spécifiée pour se déplacer contre la force de l'étrier à ressort (80) jusqu'à une position de relâchement, de manière à former un mécanisme de roue libre entre la zone de tête (30) et l'instrument de vissage (62), dans laquelle la lunette (60) présente une découpe (72) qui traverse toute la section transversale de cette dernière et forme l'étrier à ressort (80), dans laquelle la lunette (60) est configurée de manière à être au moins approximativement annulaire, dans laquelle la découpe (72) forme un premier crochet (178) qui fait saillie à partir de la zone de cou (40) et un second crochet (180) qui fait saillie à partir de l'étrier à ressort (80), et le premier crochet (178) et le second crochet (180) ont une première saillie (179) et une deuxième saillie (181), respectivement, qui se chevauchent mutuellement et sont configurées de manière à ce que la première saillie (179) et la seconde saillie (181) s'appuient l'une sur l'autre une fois que la déformation de l'étrier à ressort (80) résultant d'une expansion a atteint une mesure prédéterminée.

2. Clé dynamométrique (10) selon la revendication 1, **caractérisée en ce que** la came de verrouillage (90) est configurée de manière à faire partie intégrante de l'étrier à ressort (80) et à être d'une seule pièce avec celui-ci.

3. Clé dynamométrique (10) selon la revendication 1 ou 2, **caractérisée en ce que** la came de verrouillage (90) est adjacente à la découpe (72).

4. Clé dynamométrique (10) selon l'une des revendications 1 à 3, **caractérisée en ce que** la partie d'entraînement (92) est formée par une surface d'entraînement (92) qui s'étend de manière à être au moins approximativement radiale par rapport à l'axe de rotation (R) et qui est spécifiée pour interagir avec la face d'accouplement (94) de l'instrument de vissage (62) inséré, ladite surface d'entraînement (92) s'étendant de préférence de manière à être au moins approximativement radiale par rapport à l'axe de rotation (R).

5. Clé dynamométrique (10) selon l'une des revendications 1 à 4, **caractérisée en ce que** la came de verrouillage (90) présente un biseau (172) qui, vu dans la direction de serrage (D), suit la partie d'entraînement (92) et jouxte la partie d'entraînement (92), la distance dudit biseau (172) par rapport à l'axe de rotation (R) augmentant dans le sens inverse de la direction de serrage (D) et permettant le mécanisme de roue libre.

6. Clé dynamométrique (10) selon l'une des revendications 1 à 5, **caractérisée en ce que** l'étrier à ressort (80), sur son côté tourné vers l'axe de rotation (R), présente un évidement (96) qui, vu dans la direction de serrage (D), précède la surface d'entraînement (92) et jouxte la surface d'entraînement (92).

7. Clé dynamométrique (10) selon l'une des revendications 1 à 6, **caractérisée en ce que** la découpe (72), qui est de préférence formée par une fente (72) qui s'étend de manière à être au moins approximativement radiale par rapport à l'axe de rotation (R), est adjacente à la région de cou (30).

8. Clé dynamométrique (10) selon l'une des revendications 1 à 7, **caractérisée en ce que** l'étrier à ressort (80) sur le côté de la région de cou (30) qui est orienté à l'opposé du levier d'activation (50) fait saillie par rapport à ce dernier et est de préférence configuré de manière à faire partie intégrante de la région de cou (30) et à être d'une seule pièce avec cette dernière.

9. Clé dynamométrique (10) selon l'une des revendications 1 à 8, **caractérisée en ce qu'**une partie (82) de l'étrier à ressort (80) présente une section transversale réduite de manière à dimensionner la force qui est exercée par l'étrier à ressort (80) au moins approximativement dans la direction radiale sur l'instrument de vissage (62) inséré.

10. Clé dynamométrique (10) selon l'une des revendications 1 à 9, **caractérisée en ce que** la lunette (60) est formée au moins approximativement à la façon d'un anneau circulaire.

11. Clé dynamométrique (10) selon la revendication 9 avec une lunette (60) formée au moins approximativement à la façon d'un anneau circulaire, **caractérisée en ce que** qu'une tangente à la face circonférentielle de l'étrier à ressort (80) qui fait face à l'axe de rotation (R) en un point (P1) de la portion (82) où l'étrier à ressort (80), mesuré dans la direction radiale, a au moins approximativement la plus petite section transversale, s'étend de manière à être au moins approximativement parallèle à un rayon qui va de l'axe de rotation (R) à la partie d'entraînement (92).

12. Clé dynamométrique (10) selon l'une des revendications 1 à 11, **caractérisée par** une zone de manche (100) qui fait saillie à partir de la zone de cou (40) sur le côté de ladite zone de cou (40) qui est orienté à l'opposé de la zone de tête (30), la zone de manche (100) s'étendant de manière à être au moins approximativement parallèle au plan (E), et par une zone indicatrice (110) qui fait saillie d'une extrémité libre de la zone de manche (100), le levier d'activation (50) s'étendant au moins jusqu'à la région de l'indicateur (110), de préférence jusque dans la région de l'indicateur (110), et de façon la plus préférée au-delà de cette dernière.

13. Clé dynamométrique (10) selon la revendication 12, **caractérisée en ce que** le levier d'activation (50) est flexible, de préférence flexible de manière linéaire-élastique, et que, lorsqu'une force dans la direction de serrage (D) est appliquée à une région d'extrémité libre du levier d'activation (50), une déflexion du levier d'activation (50) à partir de la position de repos de ce dernier, est une mesure du couple généré.

14. Clé dynamométrique (10) selon la revendication 13, **caractérisée en ce que** des repères de mesure (119) sur lesquels la déviation du levier d'activation (50) peut être lue en tant que couple généré sont fixés à la zone indicatrice (110).
